# EUROPEAN PATENT APPLICATION

(11) **EP 2 974 653 A1**
(43) Date of publication of application: **20.01.2016**
(21) Application number: 14765221.8
(22) Date of filing: 14.03.2014
(51) Int. Cl.: A61B 5/12, H04R 25/00

(54) **HEARING TEST PROVISION SYSTEM, AND HEARING TEST PROVISION METHOD**

(30) Priority: 15.03.2013 JP 2013054002
(71) Applicant: Nitto Denko Corporation, Ibaraki-shi, Osaka 567-8680 (JP)
(72) Inventor: MORITA, Shigenori, Ibaraki-shi Osaka 567-8680 (JP); HAYAMA, Hideki, Ibaraki-shi Osaka 567-8680 (JP); BAN, Naoki, Ibaraki-shi Osaka 567-8680 (JP); MIMOTO, Akiko, Ibaraki-shi Osaka 567-8680 (JP); MATSUSHIMA, Ryouichi, Ibaraki-shi Osaka 567-8680 (JP); MASAKI, Toshiaki, Ibaraki-shi Osaka 567-8680 (JP); KUSUURA, Takahisa, Yokohama-shi Kanagawa 225-0011 (JP)
(74) Representative: Müller-Boré & Partner Patentanwälte PartG mbB
(86) International application number: PCT/JP2014/056993
(87) International publication number: WO 2014/142327

(57) **Abstract**

One object of the present invention is to provide a hearing test provision system capable of causing a person who has decreased hearing ability to be aware of the state in a natural way and, in consequence, capable of promoting the sale of hearing aids. The present invention contains a product presenting unit that presents products, an operating unit with which a product is selected from products presented on the product presenting unit, a database that records hearing aid affinity information which indicates affinity between a product selected by the operating unit and a hearing aid, a determining unit that determines whether to perform a hearing test on the basis of at least the hearing aid affinity information, and a hearing test conducting unit that conducts a hearing test on the basis of a determination result from the determining unit.

## Description

### TECHNICAL FIELD

The present invention relates to a hearing test provision system for supporting the sale of hearing aids and to a hearing test provision method in which the system is used.

### BACKGROUND ART

A hearing aid is an important accessory for a hearing-impaired person (a person hard of hearing) to live a comfortable life. When a person does not wear a hearing aid despite the necessity of wearing a hearing aid, symptoms of hardness of hearing progress, and the person may lose language recognition ability when the symptoms of hardness of hearing progress too far. Even if the person uses a hearing aid after being in such a state, the person may not understand words although being capable of recognizing sound. Therefore, it is important to start wearing a hearing aid at an appropriate time.

Regarding this matter, hearing aid makers and stores provide various opportunities so as to be capable of providing each person hard of hearing with an appropriate hearing aid early. For example, hearing consultations are held for people who worry about the hearing ability, and simple hearing diagnostic tests are provided on websites so as to be used freely. In addition, for example, there is suggested a hearing aid sales support system that uses the Internet (refer to Patent Document 1).

### BACKGROUND ART DOCUMENTS

### PATENT DOCUMENTS

Patent Document 1: JP-A-2002-259714

### SUMMARY OF INVENTION

### PROBLEMS THAT INVENTION IS TO SOLVE

When a person is aware of hardness of hearing with his/her ears in daily life, the person may go to an otological clinic to receive a diagnosis, may visit a hearing aid store to have a consultation, or may conduct a hearing diagnostic test on a website. However, since decrease in hearing ability progresses gradually, it is difficult to realize in an initial state. Therefore, even if an unaware person sees a leaflet about a hearing consultation meeting or items of a hearing diagnostic test on a website, because of unawareness, the person is not motivated to attend or to conduct them. In consequence, the degree of hardness of hearing may increase in most cases.

An object of the present invention is to provide a hearing test provision system and a hearing test provision method capable of causing a person who has decreased hearing ability to be aware of the state by way of a passive opportunity and, in consequence, capable of promoting the sale of hearing aids.

### MEANS FOR SOLVING PROBLEMS

In order to solve the above problem, the hearing test provision system according to the present invention contains:
a product presenting unit that presents products;
an operating unit with which a product is selected from products presented on the product presenting unit;
a database that records hearing aid affinity information which indicates affinity between a product selected by the operating unit and a hearing aid;
a determining unit that determines whether to perform a hearing test on the basis of at least the hearing aid affinity information; and
a hearing test conducting unit that conducts a hearing test on the basis of a determination result from the determining unit.

Also, the hearing test provision system according to the present invention contains:
an information presenting unit that presents information;
an operating unit with which information to browse is selected from information presented on the information presenting unit;
a determining unit that determines whether to perform a hearing test on the basis of at least information selected by the operating unit; and
a hearing test conducting unit that conducts a hearing test on the basis of a determination result from the determining unit.

Also the hearing test provision system according to the present invention contains:
an information presenting unit that presents information;
a setting unit that sets at least one of volume, tuning, and a character size at the time of presenting information on the information presenting unit;
a determining unit that determines whether to perform a hearing test on the basis of at least setting information set by the setting unit; and
a hearing test conducting unit that conducts a hearing test on the basis of a determination result from the determining unit.

Also the hearing test provision system according to the present invention contains:
an information presenting unit that presents information;
an operating unit with which information presented on the information presenting unit is operated;
an operation information obtaining unit with which operation information is obtained at the time of operating information presented on the information presenting unit with the operating unit;
a determining unit that determines whether to perform a hearing test on the basis of at least the operation information; and
a hearing test conducting unit that conducts a hearing test on the basis of a determination result from the determining unit.

Also the hearing test provision system according to the present invention contains:
an information presenting unit that presents information;
an operating unit with which information presented on the information presenting unit is operated;
an operator information obtaining unit with which operator information that indicates an operator who operates information presented on the information presenting unit with the operating unit is obtained;
a determining unit that determines whether to perform a hearing test on the basis of at least the operator information; and
a hearing test conducting unit that conducts a hearing test on the basis of a determination result from the determining unit.

It is preferable that the hearing test provision system according to the present invention further contains a visit date presenting unit that presents a proposed visit date on which a hearing aid salesperson is available for a visit when, in consequence of the hearing test, it is determined that a hearing aid is necessary.

Further, the hearing test provision method according to the present invention contains:
a product presenting step of presenting products;
a selected product information receiving step of receiving selected product information that indicates a product selected from products presented in the product presenting step;
a test conduct determining step of determining whether to perform a hearing test on the basis of at least hearing aid affinity information that indicates affinity between a product indicated by the selected product information and a hearing aid; and
a hearing test conducting step of performing a hearing test when it is determined through the test conduct determining step that a hearing test is performed.

Also the hearing test provision method according to the present invention contains:
an information presenting step of presenting information;
a browsing information receiving step of receiving browsing information that indicates browsed information among information presented in the information presenting step;
a test conduct determining step of determining whether to perform a hearing test on the basis of at least the browsing information; and
a hearing test conducting step of performing a hearing test when it is determined through the test conduct determining step that a hearing test is performed.

Also the hearing test provision method according to the present invention contains:
an information presenting step of presenting information;
a setting step of setting at least one of volume, tuning, and a character size at the time of presenting information in the information presenting step;
a test conduct determining step of determining whether to perform a hearing test on the basis of at least setting information that is set in the setting step; and
a hearing test conducting step of performing a hearing test when it is determined through the test conduct determining step that a hearing test is performed.

Also the hearing test provision method according to the present invention contains:
an information presenting step of presenting information;
an operating step of operating information presented in the information presenting step;
an operation information obtaining step of obtaining operation information that indicates the content of an operation at the time of operating information presented in the information presenting step through the operating step;
a test conduct determining step of determining whether to perform a hearing test on the basis of at least the operation information; and
a hearing test conducting step of performing a hearing test when it is determined through the test conduct determining step that a hearing test is performed.

Also the hearing test provision method according to the present invention contains:
an information presenting step of presenting information;
an operating step of operating information presented in the information presenting step;
an operator information obtaining step of obtaining operator information that indicates an operator who operates information presented in the information presenting step through the operating step;
a test conduct determining step of determining whether to perform a hearing test on the basis of at least the operator information; and
a hearing test conducting step of performing a hearing test when it is determined through the test conduct determining step that a hearing test is performed.

It is preferable that the hearing test provision method according to the present invention further contains a visit date presenting step of presenting a proposed visit date on which a hearing aid salesperson is available for a visit when, in consequence of the hearing test, it is determined that a hearing aid is necessary.

### ADVANTAGEOUS EFFECTS OF INVENTION

According to the present invention, it is possible to allow a person who is unaware of weakened hearing ability to take part in a hearing test in a natural way without giving uncomfortable feelings, and it is possible to allow the person to be aware of his/her weakened hearing ability. In consequence, it is possible to provide a hearing aid to a customer at an initial stage of decreased hearing ability, and it is possible to suppress a loss of language recognition ability (communication ability) due to a severe extent of hardness of hearing. In addition, a seller side can efficiently sell a hearing aid and in consequence, can provide an inexpensive hearing aid.

### BRIEF DESCRIPTION OF DRAWINGS

[FIG. 1] It is a diagram illustrating a configuration when the hearing test provision system according to the present invention is used in TV shopping.
[FIG. 2] It is a flowchart illustrating an example of a process procedure of the hearing test provision system illustrated in FIG. 1.
[FIG. 3] It is a diagram illustrating a configuration when the hearing test provision system according to the present invention is used in interactive TV shopping.
[FIG. 4] It is a diagram illustrating a configuration when the hearing test provision system according to the present invention is used in online shopping.
[FIG. 5] It is a diagram illustrating a configuration when the hearing test provision system according to the present invention is used at a movie theater or the like.

### MODES FOR CARRYING OUT THE INVENTION

Hereinafter, an example of embodiments of a hearing test provision system and a hearing test provision method according to the present invention will be described on the basis of the appended drawings.

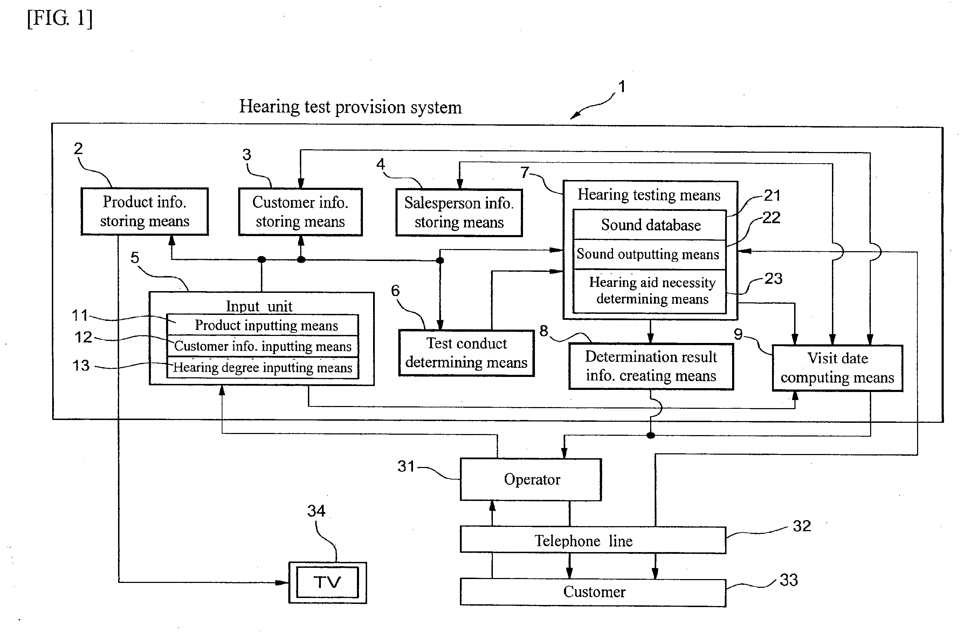

FIG. 1 is a diagram illustrating a configuration of a hearing test provision system 1. FIG. 1 illustrates a case where the hearing test provision system 1 is used in TV shopping.

The hearing test provision system 1 contains a product information storing unit 2, a customer information storing unit 3, a salesperson information storing unit 4, an input unit 5, a test conduct determining unit 6, a hearing testing unit 7, a determination result information creating unit 8, and a visit date computing unit 9. Transmission and reception of information between each means and each unit, although not illustrated, are controlled by a main operating device (CPU). Temporary storing means (RAM) is used as a work area of the main operating device.

The product information storing unit (an example of a database) 2 is a storing unit on which information of each product sold in TV shopping is stored. Product information includes, for example, salableness information of products, stock information of each product, and hearing aid affinity information of each product. The hearing aid affinity information is information defined as the degree of affinity between each product and a hearing aid and is information stored in a database in view of whether the probability that a person using a hearing aid uses the product is high. For example, a product that is expected to be used mostly by relatively aged people, such as a sphygmomanometer and reading glasses, is determined to have a high probability of being used by a person who uses a hearing aid and is determined to have a high affinity with a hearing aid. Meanwhile, a product that is expected to be used by relatively young people, such as a game console and a personal computer, is determined to have a low probability of being used by a person who uses a hearing aid and is determined to have a low affinity with a hearing aid. Hearing aids referred to herein include products such as a sound collector and an assistive hearing device in addition to hearing aids recognized as medical devices.

The customer information storing unit 3 is a storing unit on which information of a customer 33 in TV shopping is stored. The information includes, of the customer 33, name, address, telephone number, age, sex, product purchase history, product of interest, hearing aid purchase history, and an age and the like of the customer estimated from a product for purchase and the like.

The salesperson information storing unit 4 is a storing unit on which information of a hearing aid salesperson such as name, responsible territory, and schedule is stored.

The input unit 5 is an operating unit with which an operator 31 inputs information in TV shopping, and which includes a product inputting unit 11, a customer information inputting unit 12, and a hearing degree inputting unit 13.

The product inputting unit (an example of operating unit) 11 is an unit with which information of a product that the customer 33 wishes to purchase or of a product of interest (browsed product) although it does not come to the extent of purchase thereof is input into the system. The information of these products for purchase and the like that is input into the product inputting unit 11 is transmitted to the product information storing unit 2, the customer information storing unit 3, and the test conduct determining unit 6.

The customer information inputting unit 12 is a unit with which information of a product purchaser is input into the system. The information of a product purchaser that is input into the customer information inputting unit 12 is transmitted to the customer information storing unit 3 and the visit date computing unit 9.

The hearing degree inputting unit 13 is a unit with which the operator 31 who determines the degree of hearing ability of the customer 33 from a conversation exchanged between the customer 33 who makes a call and the operator 31 inputs the determination result into the system. The information of the determination result that is input into the hearing degree inputting unit 13 is transmitted to the customer information storing unit 3 and the test conduct determining unit 6.

In TV shopping, a keyboard, a touch panel, a microphone for audio input, a motion sensor for gesture input, and the like can be used as the product inputting unit 11 and the customer information inputting unit 12.

The test conduct determining unit (an example of determining unit) 6 determines whether to conduct a hearing test with respect to the customer 33 who makes access through a telephone line 32 in TV shopping. The test conduct determining unit 6 determines whether to perform a hearing test on the basis of information of the customer 33 who wishes to purchase a product.

The information of the customer 33 in a determination of whether to perform a hearing test includes, for example, information related to a product that the customer 33 wants to purchase and a product of interest to the customer, which are input by the operator 31 through the product inputting unit 11. In addition, the information includes, for example, the information of a product purchaser that is input by the operator 31 through the customer information inputting unit 12. In addition, the information includes, for example, hearing degree information of the customer 33 that is input by the operator 31 through the hearing degree inputting unit 13. Furthermore, the information includes, for example, information of a hearing aid purchase history of the customer 33 among the information related to the customer 33 stored on the customer information storing unit 3.

The determination result from the test conduct determining unit 6 is transmitted to the hearing testing unit 7.

The hearing testing unit (an example of hearing test conducting unit) 7 conducts a hearing test on the basis of the determination result of the test conduct determining unit 6. The hearing testing unit 7 conducts a hearing test with respect to the customer 33 who inform his/her wish to purchase a product through the telephone line 32. The hearing testing unit 7 includes a sound database 21, a sound outputting unit 22, and a hearing aid necessity determining unit 23.

The sound database 21 stores data of sound that is used at the time of a hearing test. The sound data serves as reference for determining whether the customer 33 is in a state where a hearing aid is necessary, that is, whether the hearing ability of the customer 33 reaches a certain level.

The sound outputting unit 22 is a unit that outputs a hearing test sound at the time of a hearing test. A speaker, for example, can be used as the sound outputting unit 22. The sound data stored on the sound database 21 is output as a hearing test sound from the speaker. The sound data that is output is transmitted to the customer 33 through the telephone line 32.

The hearing aid necessity determining unit 23 determines, on the basis of the result of a hearing test, whether the customer 33 is in a state where a hearing aid is necessary. The hearing aid necessity determining unit 23 compares the sound data output from the sound outputting unit 22 with the result of a hearing test and determines whether the hearing ability of the customer 33 reaches a certain level.

The determination result information creating unit 8 creates determination result information of a hearing test to be fed back to the customer 33 on the basis of the determination made by the hearing aid necessity determining unit 23. The created determination result information is notified to the customer 33 by the operator 31 through the telephone line 32.

The visit date computing unit (an example of visit date presenting unit) 9 computes a visit date and time of a hearing aid salesperson with respect to the customer 33. The visit date computing unit 9 computes the visit date and time when the determination result from the hearing aid necessity determining unit 23 indicates a determination that a hearing aid is necessary for the customer 33. The visit date and time is computed on the basis of the information of the customer 33 and the information of the hearing aid salesperson. Specifically, a salesperson is selected on the basis of the address of the customer 33 and the responsible territory information of a salesperson, and an available date and time for a visit is computed from information of an open schedule and information of previous and next visit locations of the salesperson. The computed visit date and time is notified to the customer 33 by the operator 31 through the telephone line 32.

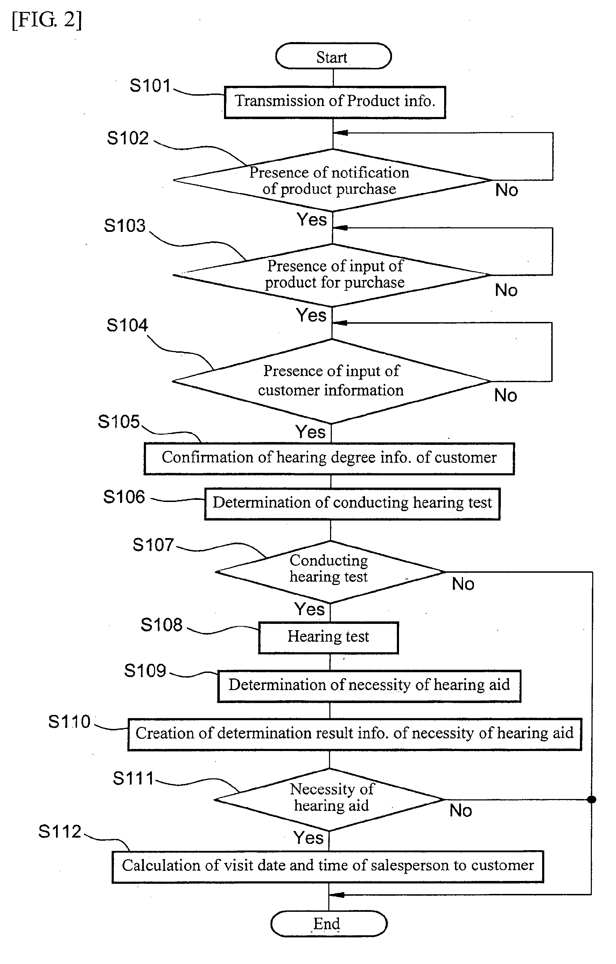

Next, an operational procedure of the hearing test provision system 1 in TV shopping will be described on the basis of FIG. 2.

Information of a product provided from the seller side in TV shopping is broadcast (transmitted) on the customer 33 side through a TV screen (an example of product presenting unit) 34 from the hearing test provision system 1 (step S101).

The hearing test provision system 1 performs a determination of whether a notification related to product purchase and the like is made from the customer 33 side (step S102). A notification from the customer 33 is transmitted to the operator 31 through the telephone line 32. When there is a call from the customer 33, the fact that a notification is made from the customer 33 is input into the input unit 5 of the hearing test provision system 1 by the operator 31.

In step S102, when there is not a notification of product purchase and the like (in the case of No), the hearing test provision system 1 goes into a notification waiting state. Meanwhile, when there is a notification of product purchase and the like (Yes in step S102), a determination of whether a product that the customer 33 wants to purchase and a product of interest are input from the input unit 5 (product inputting unit 11) is performed (step S103).

Input into the product inputting unit 11 is performed by the operator 31. The operator 31 hears (obtains) a product that the customer 33 wishes to purchase and the like via telephone. The obtained information about the product that he/she wishes to purchase and the like is input from the product inputting unit 11 by the operator 31. The input information is stored on the product information storing unit 2 and the customer information storing unit 3.

In step S103, when there is not an input of a product for purchase (in the case of No), the hearing test provision system 1 goes into a product purchase input waiting state. Meanwhile, when there is an input of a product for purchase (Yes in step S103), a determination of whether information of the customer is input from the input unit 5 (customer information inputting unit 12) is performed (step S104).

Input into the customer information inputting unit 12 is performed by the operator 31. The operator 31 hears (obtains) the information of the customer via telephone. The information of the customer includes, of the customer 33, name, address, telephone number, age, sex, product purchase history, and an age and the like of the customer estimated from the product purchase history. The age of the customer estimated from the product purchase history referred to herein means the customer age that is estimated to purchase the product and is an age that is predetermined for each product. The obtained information of the customer is input from the customer information inputting unit 12 by the operator 31. The input information is stored on the customer information storing unit 3. When the customer 33 is a customer who has a product purchase history, the information of the customer information storing unit 3 is updated.

In step S104, when there is not an input of the information of the customer (in the case of No), the hearing test provision system 1 goes into a customer information input waiting state. Meanwhile, when there is an input of the information of the customer (Yes in step S104), a confirmation of whether hearing degree information with respect to the customer 33 is input from the input unit 5 (hearing degree inputting unit 13) is performed (step S105).

Input of the hearing degree information into the hearing degree inputting unit 13 is performed by the operator 31. The operator 31 determines the degree of hearing ability of the customer 33 from a conversation about a purchase procedure exchanged with the customer 33 via telephone. The operator 31 confirms whether the customer 33 expresses difficulty in listening to a question and the like of the operator 31 (e.g., repeatedly asks). The result of the determination of the degree of hearing ability is output after being classified, for example, into two levels of good hearing ability and weak hearing ability. The output determination result is input from the hearing degree inputting unit 13 by the operator 31. The input hearing degree information is stored on the customer information storing unit 3.

Next, the hearing test provision system 1 performs a determination of whether to conduct a hearing test with respect to the customer 33 with the test conduct determining unit 6 (step S106).

The test conduct determining unit 6 performs a determination of whether to conduct a hearing test on the basis of, for example, the information of a product that the customer 33 wants to purchase and of a product of interest to the customer input from the product inputting unit 11. When the product that the customer 33 wants to purchase and the like are products that have high affinity with a hearing aid, it is determined that a hearing test is conducted. Meanwhile, when the product that the customer 33 wants to purchase and the like are products that have low affinity with a hearing aid, it is determined that a hearing test is not conducted. When the product that the customer 33 wants to purchase and the like have a medium extent of affinity with a hearing aid, a determination of whether to conduct a hearing test based on this information is suspended.

Specifically, when the product that the customer 33 wants to purchase and the like are those expected to be used mostly by relatively aged people, such as a sphygmomanometer or reading glasses, the customer 33 is determined to have a high probability of using a hearing aid, and thus it is determined that a hearing test is conducted. Meanwhile, when the product that the customer 33 wants to purchase and the like are those expected to be used mostly by relatively young people, such as a game console or a personal computer, the customer 33 is determined to have a low probability of using a hearing aid, and thus it is determined that a hearing test is not conducted.

When the product that the customer 33 wants to purchase and the like are those expected to be used regardless of age, such as a TV or a refrigerator, the probability that the customer 33 uses a hearing aid cannot be determined with this information only, and thus a determination of whether to conduct a hearing test is suspended.

The test conduct determining unit 6 performs a determination of whether to conduct a hearing test on the basis of, for example, the information of a product purchaser input from the customer information inputting unit 12. When the age of the purchaser (including an estimated age, the same applies hereinafter) is old in the information of the purchaser, it is determined that a hearing test is conducted. Meanwhile, when the age of the purchaser is young, it is determined that a hearing test is not conducted. When the customer 33 has a hearing aid purchase history, the content of a hearing test to be conducted is determined by referring to the time of purchase.

Further, the test conduct determining unit 6 performs a determination of whether to conduct a hearing test on the basis of, for example, the information of the degree of hearing ability of the customer 33 input from the hearing degree inputting unit 13. When the information of the degree of hearing ability of the customer 33 is determined as weak hearing ability, it is determined that a hearing test is conducted. Meanwhile, when the information of the degree of hearing ability of the customer 33 is determined as good hearing ability, it is determined that a hearing test is not conducted.

The test conduct determining unit 6 determines that a hearing test is conducted with respect to the customer 33 when the determination of the conduct of a hearing test is based on at least one piece of information of those determinations.

Next, the determination result in step S106 is discriminated (step S107). When the determination result indicates that a hearing test is not conducted, a hearing test is not performed with respect to the customer 33, and the process of the hearing test provision system 1 ends.

Meanwhile, when the determination result indicates that a hearing test is conducted, the intention is transmitted to the hearing testing unit 7, and a hearing test starts (step S108).

The hearing test provision system 1 conducts a hearing test with the hearing testing unit 7. The content of a hearing test is a known test content. The hearing testing unit 7 outputs the hearing test sound of the sound database 21 from the sound outputting unit 22. The output hearing test sound is transmitted to the customer 33 through the telephone line 32.

The customer 33 makes a response with voice or with a push sound to the hearing test. When the customer 33 responds with voice, the operator 31 inputs the content of the response into the input unit 5 of the hearing test provision system 1. When the customer 33 responds with a push sound, the push sound is input into the hearing test provision system 1. The input push sound is automatically discriminated in the hearing test provision system 1.

The response with voice or with a push sound that is input into the hearing test provision system 1 is transmitted to the hearing testing unit 7. The hearing aid necessity determining unit 23 of the hearing testing unit 7 determines the necessity of a hearing aid on the basis of the transmitted response (step S109).

Next, the hearing test provision system 1 creates a message for feeding the result of the hearing test back to the customer 33 with the determination result information creating unit 8. That is, information of the result of the determination of whether a hearing aid is necessary for the customer 33 is created (step S110). The created information of the determination result is transmitted to the customer 33 via the operator 31.

Next, the result of the determination of the necessity of a hearing aid is discriminated (step S111), and when a hearing aid is not necessary, the process of the hearing test provision system 1 ends. Meanwhile, when a hearing aid is necessary, the hearing test provision system 1 computes a date and time for a visit of a hearing aid salesperson to the customer 33 with the visit date computing unit (an example of visit date presenting unit) 9 (step S 112).

The visit date computing unit 9 receives the result of the determination of the necessity of a hearing aid from the hearing aid necessity determining unit 23. The visit date computing unit 9 selects an appropriate visiting salesperson on the basis of the address of the customer 33 stored on the customer information storing unit 3 and the responsible territory information of a salesperson stored on the salesperson information storing unit 4. Then, an available date and time for a visit are computed from the schedule of the visiting salesperson. The computed available date and time for a visit are transmitted to the customer 33 via the operator 31. When a visit date and time is determined after being confirmed by the customer 33, the determined content is input into the input unit 5 of the hearing test provision system 1 through the operator 31, and a series of processes ends.

FIG. 2 illustrates an example in which a hearing aid salesperson visits the customer 33. However, without being limited thereto, any method is acceptable provided that an opportunity for a customer and a hearing aid salesperson to have a conversation about the necessity of a hearing aid and the like can be provided. For example, a proposed visit date is computed by the visit date computing unit 9 from the schedules of a customer and a visiting salesperson, and an appointment for a meeting with the customer is made. Then, the customer may visit the hearing aid store and the like at the date and time of the meeting, or they may meet at a predetermined place and have the meeting.

With a configuration such as above, the hearing test provision system 1 of the present embodiment can be used in TV shopping to allow a person (customer) unaware of weakened hearing ability to take part in a hearing test in a natural way without resistance.

Hitherto, a hearing aid is of little interest to a person who is unaware of decreased hearing ability, and in such a background, a hearing aid sales system that is developed for the purpose of simply promoting the sale of only hearing aids is not efficient. Therefore, the hearing test provision system 1 of the present embodiment, when a person who does not have subjective symptoms intends to purchase another product in TV shopping, secures an opportunity of selling a hearing aid during the flow of the product purchase. During the flow of the product purchase, that is, during when a series of purchase procedures is being performed on the product, a hearing test is conducted with respect to the customer. Then, the result is fed back to the customer until the purchase procedures end, and thus it is possible to point out the weakened hearing ability without giving uncomfortable feelings to the customer. Accordingly, it is possible to allow the customer to be aware of weakened hearing ability without giving any feeling of repulsion.

In consequence, it is possible to provide a hearing aid to a customer at an initial stage of decreased hearing ability, and it is possible to prevent a loss of language recognition ability (communication ability) due to a severe extent of hardness of hearing.

There is a psychologically high hurdle for a person who does not have subjective symptoms related to weakened hearing ability to go to a hearing aid store and the like, and it is difficult for him/her to take the action immediately. Therefore, the hearing test provision system 1 of the present embodiment points out weakened hearing ability and further makes it possible to propose, on site, the visit of a visiting salesperson to a home of the customer and the like and the visit date. Accordingly, it is possible to remove the psychologically high hurdle such that the customer goes to the store to purchase a hearing aid, and this can lead to promoting the sale of hearing aids. Apparently, the customer may decide whether a visiting salesperson visits the home of the customer. The customer may go to the store from the customer side on the basis of the informed result of a hearing test or may meet a salesperson at a specified place. At the time of a meeting with the customer, it is possible to not only sell a hearing aid but also recommend a consultation with a doctor, give simple treatment, or adjust the hearing condition and the like of a hearing aid for a customer who already possesses a hearing aid.

Furthermore, it is possible to obtain an opportunity of selling a hearing aid with respect to a customer who intends to purchase a desired product through TV shopping (an opportunity of purchasing another product other than purchasing a hearing aid). Since the customer already decides to purchase another product, there is a psychologically low hurdle for the customer to purchase a hearing aid in addition to the product, and this can lead to more efficient selling. Thus, the seller side can efficiently sell a hearing aid and in consequence, can provide an inexpensive hearing aid.

Information related to a product that the customer wants to purchase or to a product of interest (affinity with a hearing aid), information of the customer such as age of the customer, information of the degree of hearing ability of the customer that is determined through a conversation with the customer, or the like is referred to at the time of a determination of whether to conduct a hearing test with respect to the customer. Thus, it is possible to efficiently find a customer for whom a hearing aid is highly necessary.

For example, it is possible to efficiently conduct a hearing test with respect to a customer who has decreased hearing ability by conducting a hearing test with respect to only a customer who intends to purchase a product that has high affinity with a hearing aid, and thus it is possible to promote the sale of hearing aids. Similarly, it is possible to efficiently sell a hearing aid by, for example, not conducting a hearing test in the case of a product of which the purchase rate is low in an age group having weakened hearing ability and performing a hearing test in the case of a product of which the purchase rate is high for middle-aged and elderly people. Similarly, it is possible to sell a hearing aid more efficiently by conducting a hearing test with respect to only a customer who is determined to have a low degree of hearing ability.

A hearing test is not performed for a customer who already has a hearing aid purchase history by referring to a purchase history of the customer. Accordingly, it is possible to prevent conducing the same hearing test repeatedly for the customer, and it is possible to prevent a decrease in the purchase intention of the customer. Furthermore, when a certain period of time passes from the previous purchase of a hearing aid, it is possible to provide aftersales service such as the maintenance of the hearing aid by performing a hearing test that is different from the previous one and confirming the condition.

### <Modification Example>

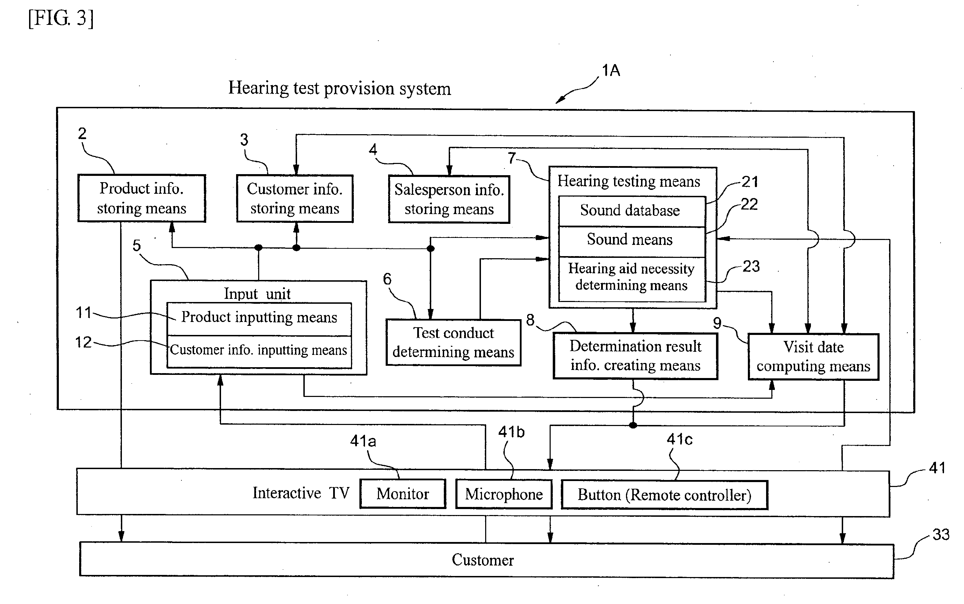

FIG. 3 illustrates a first modification example of the hearing test provision system 1 (hearing test provision system 1A). FIG. 3 illustrates the case where the hearing test provision system 1A is used in interactive TV shopping that is combined with the Internet. In the description below, the same or corresponding part in comparison with the hearing test provision system 1 will be designated by the same reference sign, and a description thereof will be appropriately omitted.

In an interactive TV shopping, the customer 33 purchases a product that he/she wishes to purchase by using an interactive function of digital broadcasting. The customer 33 performs a purchase procedure for a product that he/she wants to purchase via a microphone 41 b, a button (remote controller) 41 c, a motion sensor for a gesture input, or the like while viewing a monitor 41a through an interactive TV (an example of product presenting unit) 41. Information transmitted through the microphone 41 b or through the button (remote controller) 41c is input into the input unit 5 of the hearing test provision system 1A. Transmission and reception of information between the customer side and the seller side (hearing test provision system 1A) are performed through an Internet line that is connected to the interactive TV 41.

In a hearing test, first, a message of prompting a customer for whom performing a test is determined as necessary to have a hearing test, such as "Please respond to a hearing test", is displayed on the monitor 41 a of the interactive TV 41. A hearing test is conducted only when the customer accepts. The customer responds via the microphone 41 b or the button (remote controller) 41c to the hearing test sound that is output from a speaker of the interactive TV 41.

The necessity of a hearing aid for the customer is determined from the result of the hearing test, and the content of the determination is displayed on the monitor 41 a of the interactive TV 41 as a message fed back to the customer.

The available date and time for a visit that are computed by the visit date computing unit 9 are displayed on the monitor 41 a of the interactive TV 41. The customer 33 decides the visit date and time by using the microphone 41 b and the button (remote controller) 41c.

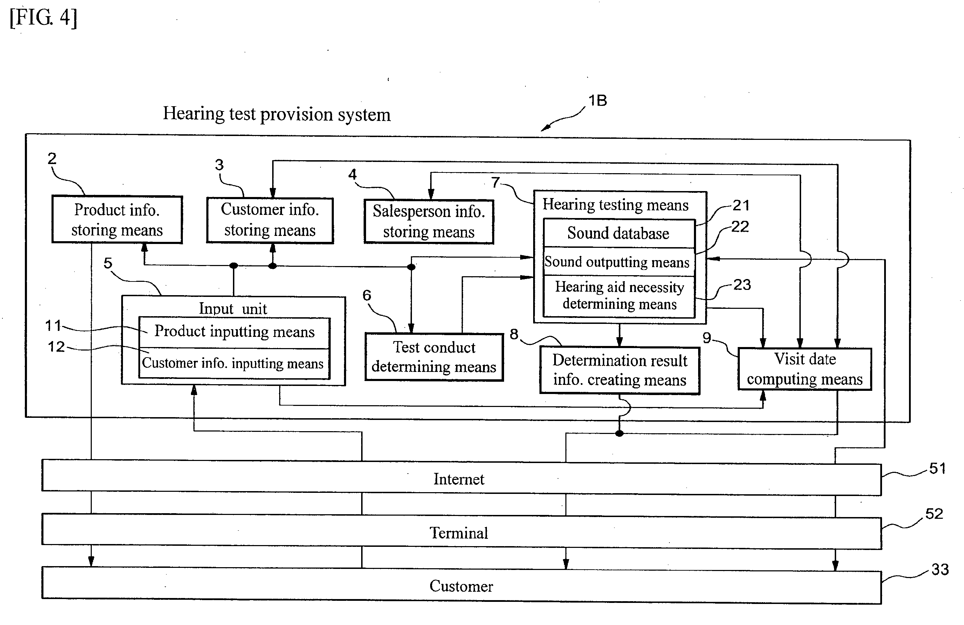

FIG. 4 illustrates a second modification example of the hearing test provision system 1 (hearing test provision system 1 B). FIG. 4 illustrates the case where the hearing test provision system 1B is used in online shopping. In the description below, the same or corresponding part in comparison with the hearing test provision system 1 will be designated by the same reference sign, and a description thereof will be appropriately omitted.

In online shopping, the seller side provides sales products to the customer side by registering on a website (an example of product presenting unit). The customer 33 purchases a product that he/she wishes to purchase on the website through the Internet 51. The customer 33 performs a purchase procedure for the product that he/she wants to purchase via a terminal (personal computer or a mobile electronic terminal) 52. Similarly, the customer 33 inputs information of the customer via the terminal 52. Information transmitted from the terminal 52 is input into the input unit 5 of the hearing test provision system 1B.

In a hearing test, first, a message of prompting a customer for whom performing a test is determined as necessary to have a hearing test, such as "Please respond to a hearing test", and "start button" for a hearing test and the like are displayed on a screen of the terminal 52. A hearing test is conducted only when the customer accepts and presses "start button". The customer responds to the hearing test sound that is output from a speaker of the terminal 52 by directly inputting or selecting an option on a response input screen displayed on the screen of the terminal 52.

The necessity of a hearing aid is determined from the hearing test, and the content of the determination is displayed on the screen of the terminal 52 as a message fed back to the customer.

The available date and time for a visit that are computed by the visit date computing unit 9 are displayed on the screen of the terminal 52. The customer 33 decides the visit date and time by operating a button of the terminal 52.

Although illustration is omitted, it is possible to provide a configuration, as a third modification example, in which a hearing test provision system 1C is used at a storefront. Sales products are lined up and provided at the storefront. The customer 33 performs a purchase procedure for a product that he/she wants to purchase through a salesperson at the storefront. Similarly, the customer 33 transmits information thereof to the storefront salesperson. The storefront salesperson inputs the information of the customer 33 (information of the product that he/she wants to purchase, information of the customer, and information of the degree of hearing ability of the customer determined by the storefront salesperson) from a terminal of the hearing test provision system 1C.

A hearing test is conducted on a hearing test terminal that is connected via communicating unit to the hearing test provision system 1C, and the result is transmitted to the hearing test provision system 1C.

The necessity of a hearing aid is determined from the hearing test, and a feedback message to the customer 33 is displayed on the terminal. The message is transmitted to the customer by the storefront salesperson.

The available date and time for a visit computed by the visit date computing unit 9 are displayed on a screen of the terminal, and the customer decides the visit date and time with the storefront salesperson.

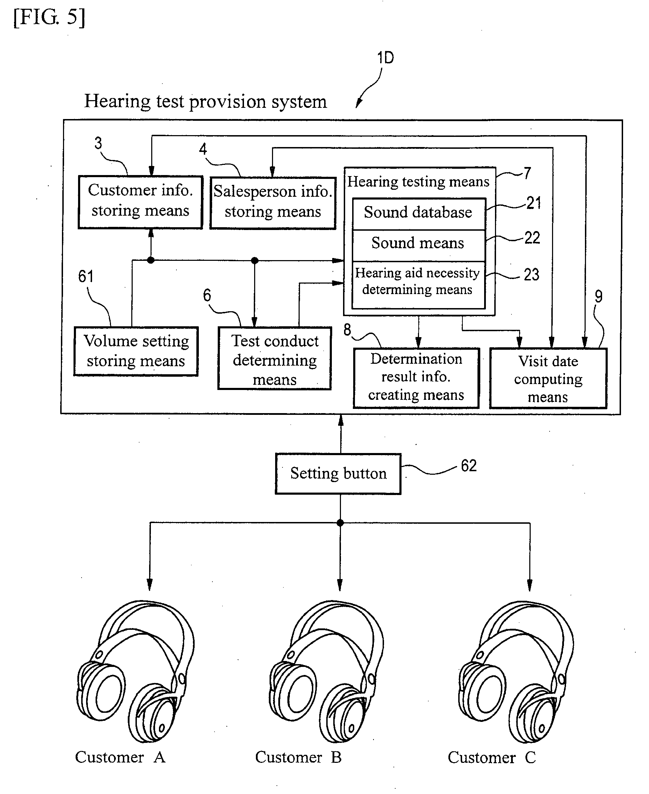

FIG. 5 illustrates a fourth modification example of the hearing test provision system 1 (hearing test provision system 1D). FIG. 5 illustrates the case where the hearing test provision system 1D is used at a movie theater, an art gallery, a lecture presentation, and the like. In the description below, the same or corresponding part in comparison with the hearing test provision system 1 will be designated by the same reference sign, and a description thereof will be appropriately omitted.

At a movie theater, an art gallery, a lecture presentation, and the like, each viewer (customer) has an opportunity of listening to the explanation of the content of a work by wearing a headphone (an example of information presenting unit). The hearing test provision system 1D estimates the necessity of a hearing aid from the set level of the volume, the set content of the tuning, and the like of the headphone with which the customer listens to the explanation, and guides the customer to conduct a hearing test.

The headphone used by each customer (customer A, customer B, and customer C) is connected to a server, and the set level of the volume, the set content of the tuning, and the like of the headphone used by each customer are transmitted to the server. The customer sets the volume and the like of the headphone by using a corresponding setting button (an example of setting unit) 62. Each setting information (volume setting information) of the headphone transmitted is stored on volume setting storing unit 61 of the hearing test provision system 1 D.

The test conduct determining unit 6 determines whether to conduct a hearing test on the basis of the set level of the volume, the set content of the tuning, and the like stored on the volume setting storing unit 61. For example, when the set level of the volume is set to be greater than or equal to a predetermined level, the customer is determined as the target of a hearing test.

The determination result from the test conduct determining unit 6 is transmitted to the hearing testing unit 7. The hearing testing unit 7 performs a test only with respect to a customer for which it is determined that conducting a hearing test is necessary. The hearing testing unit 7 transmits the hearing test sound to the headphone that is used by the customer who is the target of the conduct of the hearing test.

The customer responds to the hearing test by using an operating button that is disposed on each viewer seat or on each headphone.

Information of the result of the hearing test may be transmitted to each customer through the headphone or may be directly transmitted verbally.

Next, a fifth modification example of the hearing test provision system 1 (hereinafter, referred to as a hearing test provision system 1 E) will be described. The hearing test provision system 1E determines whether to conduct a hearing test with respect to the customer on the basis of information that is not correlated with the purchase of a product and that is not directly related to a hearing aid, and determines the necessity of a hearing aid for the customer from the result of the hearing test.

For example, an age of a browser (customer) is estimated on the basis of history information of the website browsed with a personal computer, a smartphone or the like (an example of information presenting unit), and the necessity of a hearing test is determined.

The hearing test provision system 1 E is provided with age estimating unit that estimates an age of a person (browser) who accesses the website. The age estimating unit estimates an age of the customer by analyzing information of the browser that remains on a web server when the website is browsed and his/her browsing tendency.

The analysis of the browsing tendency and the estimation of the age by the age estimating unit can be specifically performed as follows.

Points are given in advance to each information that the customer can browse on the website. Giving points is performed by weighing information in which elderly people tend to show interest. For example, when there is information related to "grandchild", 95 points are given to the information because the probability that elderly people show interest therein is high. Similarly, points are given on the basis of interest of elderly people such as 50 points for information related to "pension" because not only elderly people but also young people may probably show interest therein, 85 points for information related to "game of go", 80 points for "shogi", 70 points for "sumo", 20 points for "soccer", and 5 points for "a game console".

Multiple pieces of browsing history information are collected for each customer. For example, when the total points of 10 pieces of collected browsed information exceed predetermined points that are set in advance, the age of the customer is estimated to exceed, for example, 60, and the customer is determined as a target for whom a hearing test is necessary. In addition, when the average value of the points of browsed information exceeds predetermined points that are set in advance, he/she may be determined as a target for whom a hearing test is necessary in the same manner.

The hearing test provision system 1E may be a system that, for example, estimates the age of the customer from information of an image of the customer obtained through a TV telephone, an image of the customer captured by a web camera, and the like, and determines the necessity of a hearing test.

Information is presented to the customer via a personal computer, a TV telephone, or the like (an example of information presenting unit) through the Internet. Information of an image of the customer who operates the personal computer, TV telephone, or the like is obtained by using a web camera, a camera of the TV telephone (including a smartphone and a tablet terminal), or the like (an example of operator information obtaining unit). A control unit (an example of operation information obtaining unit) of the personal computer or the like analyzes information of the face of the customer from the obtained image, information of the fingers that operate a smartphone, tablet terminal, keyboard, mouse, or the like, and set information (character size and volume) in an electronic device such as a smartphone. Then, the age of the customer is estimated on the basis of the texture and glossiness of the face, the fingers, and the like of the analyzed customer, the input speed on a smartphone, tablet terminal, and keyboard, the operating speed on a mouse, the content of settings in an electronic device, and the like. The hearing test provision system 1E determines the necessity of a hearing test from the estimated age of the customer and determines the necessity of a hearing aid from the result of the hearing test.

Furthermore, an odor sensor may be installed in a TV telephone, a web camera, or the like so as to sense an aging odor that has a high probability of occurring in elderly people. The necessity of a hearing test is determined by analyzing the sensed aging odor and estimating the age of the customer. Then, the necessity of a hearing aid may be determined from the result of the hearing test.

The distance between a screen and a user may be measured by using an image from a TV camera, a web camera, or a camera of a smartphone or by using a distance measuring sensor, and the age may be estimated according to the distance. For example, the age may be estimated according to the distance by assuming that a person moves the face thereof close to the screen when having decreased eyesight.

The hearing test provision system 1E may be one in which, for example, a hearing aid salesperson and the like carry out a questionnaire for the customer that is not directly related to a hearing aid, the age of the customer is estimated on the basis of the result of selection in the questionnaire, and the necessity of a hearing test is determined.

The content of a hearing test and the content of a determination of the necessity of a hearing aid from the result of the hearing test are the same as those of the hearing test provision system 1.

The hearing test provision systems 1A, 1 B, 1C, 1D, and 1E of the modification examples described above can accomplish the same effect as the hearing test provision system 1.

While the present invention is described in detail with reference to a certain embodiment, it is apparent for those skilled in the art that various modifications and changes can be added thereto without departing from the spirit and the scope of the present invention.

The present application is based upon Japanese Patent Application No. 2013-054002, filed on March 15, 2013, the content of which is incorporated herein by reference.

### DESCRIPTION OF REFERENCE NUMERALS AND SIGNS

- 1, 1A, 1 B, 1C, 1D:: Hearing test provision system
- 2:: Product information storing unit (example of database)
- 3:: Customer information storing unit
- 4:: Salesperson information storing unit
- 5:: Input unit
- 6:: Test conduct determining unit (example of determining unit)
- 7:: Hearing testing unit (example of hearing test conducting unit)
- 8:: Determination result information creating unit
- 9:: Visit date computing unit
- 11:: Product inputting unit (example of operating unit)
- 12:: Customer information inputting unit
- 13:: Hearing degree inputting unit
- 21:: Sound database
- 22:: Sound outputting unit
- 23:: Hearing aid necessity determining unit

## Claims

1. A hearing test provision system comprising:
a product presenting unit that presents products;
an operating unit with which a product is selected from products presented on the product presenting unit;
a database that records hearing aid affinity information which indicates affinity between a product selected by the operating unit and a hearing aid;
a determining unit that determines whether to perform a hearing test on the basis of at least the hearing aid affinity information; and
a hearing test conducting unit that conducts a hearing test on the basis of a determination result from the determining unit.

2. A hearing test provision system comprising:
an information presenting unit that presents information;
an operating unit with which information to browse is selected from information presented on the information presenting unit;
a determining unit that determines whether to perform a hearing test on the basis of at least information selected by the operating unit; and
a hearing test conducting unit that conducts a hearing test on the basis of a determination result from the determining unit.

3. A hearing test provision system comprising:
an information presenting unit that presents information;
a setting unit that sets at least one of volume, tuning, and a character size at the time of presenting information on the information presenting unit;
a determining unit that determines whether to perform a hearing test on the basis of at least setting information set by the setting unit; and
a hearing test conducting unit that conducts a hearing test on the basis of a determination result from the determining unit.

4. A hearing test provision system comprising:
an information presenting unit that presents information;
an operating unit with which information presented on the information presenting unit is operated;
an operation information obtaining unit with which operation information is obtained at the time of operating information presented on the information presenting unit with the operating unit;
a determining unit that determines whether to perform a hearing test on the basis of at least the operation information; and
a hearing test conducting unit that conducts a hearing test on the basis of a determination result from the determining unit.

5. A hearing test provision system comprising:
an information presenting unit that presents information;
an operating unit with which information presented on the information presenting unit is operated;
an operator information obtaining unit with which operator information that indicates an operator who operates information presented on the information presenting unit with the operating unit is obtained;
a determining unit that determines whether to perform a hearing test on the basis of at least the operator information; and
a hearing test conducting unit that conducts a hearing test on the basis of a determination result from the determining unit.

6. The hearing test provision system according to any one of Claims 1 to 5, further comprising:
a visit date presenting unit that presents a proposed visit date on which a hearing aid salesperson is available for a visit when, in consequence of the hearing test, it is determined that a hearing aid is necessary.

7. A hearing test provision method comprising:
a product presenting step of presenting products;
a selected product information receiving step of receiving selected product information that indicates a product selected from products presented in the product presenting step;
a test conduct determining step of determining whether to perform a hearing test on the basis of at least hearing aid affinity information that indicates affinity between a product indicated by the selected product information and a hearing aid; and
a hearing test conducting step of performing a hearing test when it is determined through the test conduct determining step that a hearing test is performed.

8. A hearing test provision method comprising:
an information presenting step of presenting information;
a browsing information receiving step of receiving browsing information that indicates browsed information among information presented in the information presenting step;
a test conduct determining step of determining whether to perform a hearing test on the basis of at least the browsing information; and
a hearing test conducting step of performing a hearing test when it is determined through the test conduct determining step that a hearing test is performed.

9. A hearing test provision method comprising:
an information presenting step of presenting information;
a setting step of setting at least one of volume, tuning, and a character size at the time of presenting information in the information presenting step;
a test conduct determining step of determining whether to perform a hearing test on the basis of at least setting information that is set in the setting step; and
a hearing test conducting step of performing a hearing test when it is determined through the test conduct determining step that a hearing test is performed.

10. A hearing test provision method comprising:
an information presenting step of presenting information;
an operating step of operating information presented in the information presenting step;
an operation information obtaining step of obtaining operation information that indicates the content of an operation at the time of operating information presented in the information presenting step through the operating step;
a test conduct determining step of determining whether to perform a hearing test on the basis of at least the operation information; and
a hearing test conducting step of performing a hearing test when it is determined through the test conduct determining step that a hearing test is performed.

11. A hearing test provision method comprising:
an information presenting step of presenting information;
an operating step of operating information presented in the information presenting step;
an operator information obtaining step of obtaining operator information that indicates an operator who operates information presented in the information presenting step through the operating step;
a test conduct determining step of determining whether to perform a hearing test on the basis of at least the operator information; and
a hearing test conducting step of performing a hearing test when it is determined through the test conduct determining step that a hearing test is performed.

12. The hearing test provision method according to any one of Claims 7 to 11, further comprising:
a visit date presenting step of presenting a proposed visit date on which a hearing aid salesperson is available for a visit when, in consequence of the hearing test, it is determined that a hearing aid is necessary.
